(19) Europäisches Patentamt
European Patent Office
Office européen des brevets

(11) Publication number: 0 424 027 A1

(12) **EUROPEAN PATENT APPLICATION**

(21) Application number: 90311149.0

(51) Int. Cl.5: C12N 15/00, C12N 15/86

(22) Date of filing: 11.10.90

The applicant has filed a statement in accordance with Rule 28 (4) EPC (issue of a sample only to an expert). Accession number(s) of the deposit(s): ATCC 68106 and 68107.

(30) Priority: 16.10.89 US 421886

(43) Date of publication of application: 24.04.91 Bulletin 91/17

(84) Designated Contracting States: CH DE FR GB IT LI NL

(71) Applicant: MERCK & CO. INC.
126, East Lincoln Avenue P.O. Box 2000
Rahway New Jersey 07065-0900(US)

(72) Inventor: Garber, Ellen A.
5 Wedgewood Lane
Holmdel, NJ 07733(US)
Inventor: Kopchick, John J.
4 Orchard Lane
Athens, Ohio 45701(US)

(74) Representative: Hesketh, Alan, Dr. et al
European Patent Department Merck & Co., Inc. Terlings Park Eastwick Road
Harlow Essex, CM20 2QR(GB)

(54) **Vectors for generating transgenic fowl.**

(57) Retroviral vectors have been produced that facilitate production of transgenic fowl. The vectors are nonpermuted constructs carrying a gene encoding a high titer polymerase. Transgenic fowl have been produced by infection of fresh fertile eggs with a recombinant retroviral vector carrying the bovine growth hormone gene. Integration and expression of the exogenous gene has resulted in the production of desirable traits in the fowl developing from such treatment.

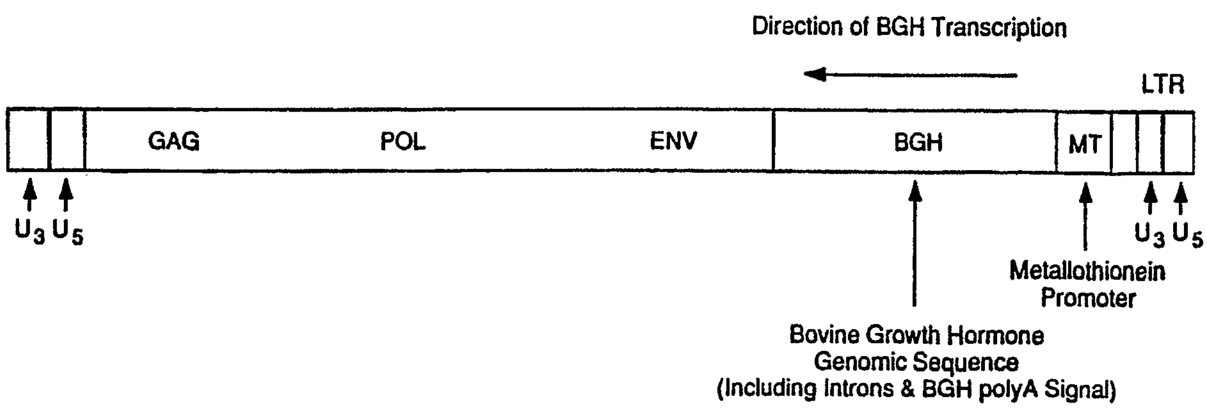


FIG. 4

EP 0 424 027 A1

# VECTORS FOR GENERATING TRANSGENIC FOWL

## BACKGROUND OF THE INVENTION

Traditional techniques for selective animal breeding are designed to enhance desired traits in the offspring. The terms "desired traits" and the like are used herein to refer to phenotypic characteristics providing a distinct commercial advantage in the animal undergoing selection. The possibilities for enhancing desirable animal traits range from inducing more efficient growth and feeding characteristics to enhanced disease resistance. Selective animal breeding is slow, labor intensive, and often does not result in the selection of a given desirable trait.

Production of transgenic animals by introduction of exogenous genes into their germline has been reported for mice, rabbits, pigs, sheep, and fish [Brinster et al., PNAS USA 82 : 4438-4442 (1985); Hammer et al., Nature 315 : 680-683 (1985); Purcell et al., Science 244 : 1281-1288 (1989)]. Whereas the ability to vaccinate livestock has been realized for a number of animal species and for a number of bacterial and viral infections, the cost, tedium, and limitation to antigens that may be safely prepared and efficaciously administered makes the newly emerging technology of genetic manipulation tremendously appealing.

Techniques used in the production of these genetically altered animals vary from microinjection of DNA into eggs to culturing of explanted bone marrow in the presence of recombinant retroviruses followed by reimplantation into the radiation-treated host. The experimental results have ranged from showing little effect to the production of mice twice the size of their untreated littermates [Palmiter et al., Nature 300 : 611 (1982); Palmiter et al., Science 222 : 809 (1983); Brinster et al., PNAS USA 82 : 4438-4442 (1985)].

The production of a fowl line with enhanced disease resistance is far more appealing than the ability to vaccinate a given fowl population. However, technology developed in the vaccination art provides promising insights as to the direction the genetic approach should take. For example, Chambers et al. [Virology 167 : 414-421 (1988)] reported protection of chickens from influenza viral infection by inoculation of the fowl with live recombinant vaccinia virus carrying the hemaglutinin gene of influenza A/Turkey/Ireland /1378/83 (H5N8). Although this study presents vaccination as a solution to influenza infection, it indicates that if the chicken itself could express the hemaglutinin gene described, then vaccination would become superfluous once an avian strain became established.

Manipulation of the avian genome has been frustrated because the single celled fertilized egg is inaccessible and the early chick embryo already has 10,000-50,000 cells [Giladi et al., Dev. Biol.49 : 321-337 (1976); Kochav et al., Dev. Biol. 79 : 296-308 (1980)]. One method that has potential to overcome this accessibility barrier is to use avian retroviruses as vectors for introduction of foreign genes into the early avian embryo. Their infectivity allows exposure of a foreign gene of interest to many cells, including germline cells. Introduction of avian retroviruses and vectors into the developing chicken egg has been reported, and development of birds with exogenous retroviral genetic material has been successful [Bosselman et al., Science 243 : 533-535 (1989); Salter et al., Virology 157 : 236-240 (1987)].

In U.S. Application Serial No. 80,278, filed July 31, 1987, Hughes et al. disclosed an avian retroviral vector. However, their system did not incorporate a foreign gene, nor was it demonstrated that their invention would in fact yield a fowl with an altered phenotype. Souza et al. [J. Exp. Virol. 232 : 465-473 (1984)] reported introduction of chicken growth hormone (cGH) gene into nine-day-old chicken embryos. The results of their study indicated a complete lack of effect of the treatment on bird growth, in spite of the fact that they reported a serum cGH concentration three-to-tenfold higher than in control animals. Peebles, et al. [Growth Dev. and Aging 52 :133-138 (1988)] reported no effect on the rate of chicken growth, even upon administration of extremely high doses of chicken growth hormone. Bosselman et al. [Science 243 : 333-535 (1989)] reported injection of a replication-defective reticuloendotheliosis virus (REV) vector into chicken eggs. Their vector was shown to be capable of infecting germline stem cells and stably incorporated the herpes simplex virus type 1 thymidine kinase gene and the Tn5 neomycin phosphotransferse gene into the chicken genome. Progeny were examined and shown to have received these extrachromosomal elements in a mendelian fashion. However, no desirable trait was noted in these fowl. Hippenmeyer et al. [Nucleic Acids Research 16 : 7619-7632 (1989)] showed that a Rous Sarcoma Virus derived replication competent vector could be used to generate fowl expressing the bacterial neomycin phosphotransferase gene. Once again, however, no desirable phenotypic effect was demonstrated in these fowl.

Crittendon et al. [Second Symposium on the Genetic Engineering of Animals, Abstract: June 25-29 (1989); U.S. application 7,217,994 filed July12, 1988] reported that one of the chickens that emerged from an egg infected with ALV subgroup A (containing no foreign gene), was observed to be non-viremic,

presumably due to deletion of required viral replicative or infective functions. When this bird was challenged with infection by ALV, the chicken was fortuitously found to be resistant. However, the strongly undesirable increased incidence of bursal lymphoma development in progeny homozygous or heterozygous for the integrated sequences was noted.

Morrison et al. [Virology 171 : 10-17 (1989)] transfected chick embryo fibroblasts in culture with a replication competent Schmidt-Ruppin Rous sarcoma virus derived vector carrying the Newcastle disease virus cDNA of the hemagglutinin-neuraminidase gene. Transfected cultured cells expressed the neuraminidase activity and were found to be resistant to infection by Newcastle disease virus. However, these researchers noted that cells transfected with a similar construct expressing the influenza virus hemagglutinin gene were not resistant to challenge by the influenza virus. Thus it is difficult to predict the effects in vivo of expressing an exogenous gene product, even if expression of that gene is apparent in vitro or in vivo. Consequently, the term "reactive expression" will be used herein to denote more than the process of an animals' generation of a given gene product, as such production of a given protein may or may not generate a transgenic with a desirable characteristic. This term is used to define a transgenic that in fact develops a phenotype improved by the expression of the given foreign gene.

Thus, no report has to our knowledge been presented wherein a gene foreign to both the retroviral vector and the avian genome has been introduced into the avian genome that conferred a desirable trait in the bird developing from such treatment. Accordingly, the insertion of the bovine growth hormone into the developing fowl egg by means of a retroviral vector would not be expected to yield any desirable effect on the rate of growth of the fowl. However, applicants disclose herewith that in fact such treatment has yielded a fowl with enhanced growth characteristics which may considerably impact the poultry industry.

Throughout the present application, the term "avian" is intended to include the various known strains of Gallus gallus, or chickens, (e.g. white leghorn, brown leghorns, Sussex, New Hampshire, Rhode Island, Ausstralorp, Minorca, Amrox, California gray, Italian partridge-colored), as well as turkeys, pheasants, quail, duck, and other poultry commonly bred in commercial quantities. This breadth is justified in that the A strain of RSV is known to infect chickens, turkeys, pheasants, red jungle fowl. In addition, in vitro infection of cultured cells for most of these species, and retroviruses whose natural host is a member of one of these species, has been demonstrated for retroviruses not unlike the RSV upon which the current vector constructions are based (see figure 1. adapted from Molecular Biology of Tumor Viruses, 2nd Edition, RNA Tumor Viruses, Cold Spring Harbor Laboratory, 1982, appendix).

The retroviral vectors of the instant invention have the additional advantages of stability, absence of oncogenic sequences, high infectivity to the target tissues of interest, nonpermuted structure therefore requiring minimal in vitro manipulation, and a gene encoding an efficient polymerase that minimizes the culture time necessary to raise a high titer of infectious virus. These and other advantages facilitate production of transgenic fowl useful in the poultry industry for breeding better chickens, turkeys, and other avian animals. Therefore, the present invention is a substantial step forward in the quest for a retroviral vector delivery system for the production of transgenic fowl.

It is an object of this invention to produce a vector capable of inserting a foreign expressible gene of interest into the avian genome. Another object of this invention is to produce a vector that is nonpermuted such that minimal in vitro manipulation is required prior to transfection of a host cell. A further object of this invention is to produce a vector that encodes a high titer polymerase such that a short interval of culture time is required to raise a high titer stock of infectious virus. Another object of this discovery is to produce a bird that carries an expressed foreign gene endowing the animal with a desirable characteristic. A further object of the invention is to produce a second generation bird expressing the same desirable characteristic expressed in its parents due to germ line insertion of the desirable gene. Additional advantages of the invention will become apparent from the complete description.

## SUMMARY OF THE INVENTION

The disclosed invention relates to the production of vectors useful for producing transgenic fowl carrying and expressing a foreign expressible gene. More specifically, replication competent and replication defective retroviral vectors have been constructed that allow expression of foreign genes in vitro and in vivo. The bovine growth hormone and the firefly luciferase genes were stably incorporated into cultured avian cells and functional gene products were expressed. In vivo, a nonpermuted retroviral vector derived from the Schmidt-Ruppin A strain of Rous Sarcoma Virus (SRA-RSV) carrying the bovine growth hormone gene under transcriptional regulation of the mouse metallothionein promoter has been introduced into the developing chicken embryo. The SRA-RSV pol gene was replaced by the Bryan High titer polymerase gene

to enable the production in vitro of a high titer stock of the viral vector for infection of the avian egg without loss of the bovine growth hormone gene. Oncogenic sequences were removed and allowed insertion of the bGH gene along with the zinc inducible metallothionein promoter. As a result of integration of the bovine growth hormone into the genome of the chickens, transgenic birds were derived which had detectable levels of circulating bovine growth hormone. Some of these fowl developed more rapidly than control chickens as measured by leg length, weight, and earlier sperm production.

## BRIEF DESCRIPTION OF THE DRAWINGS

Figure 1: Comparison of the molecular maps of the proviral DNA of a number of related avian retroviruses.

Figure 2: Strategy for construction of a nonpermuted, replication competent, retroviral vector pNPBGH85 carrying the Bryan High Titer Polymerase, the Bovine Growth Hormone Gene, and the Metallothionein Promoter.

Figure 3: Strategy for construction of a nonpermuted, replication defective retroviral vector pNPBGHdef carrying the Bryan High Titer Polymerase, the Bovine Growth Hormone Gene, and the Metallothionein Promoter.

Figure 4: Detail of the proviral DNA integrated into the chicken genome as a result of reverse transcription of infective virus generated by pNPBGH85 transfection of chicken embryo fibroblasts.

## DEFINITIONS AND ABBREVIATIONS

clone: cells in a cell line or nucleic acid molecules which are genetically identical.

expression: the process whereby the genetic code of DNA becomes transcribed into RNA and subsequently translated into protein.

foreign gene: a gene not normally found in the genome of a given virus, cell or organism.

gene: a piece of genetic material, DNA or RNA, which encodes information for a specific protein.

genome: the total genetic material composed of DNA or RNA of an organism, cell, or virus.

homologous recombination: the mechanism proposed for the observation that genetic elements having sequence homology may rearrange themselves in a biological system, incorporating segments of the original nucleic acids to yield a new genetic element.

in trans: the complementation of a defective trait by production of the missing element from a secondary source.

insert: a piece of genetic material that may be joined with another piece of genetic material
integration: the process whereby a segment of DNA becomes joined with the chromosome of a live organism or cell.

integration: the process whereby sequences from one source become incorporated into the genome of a cell or organism.

ligation: the process of joining two DNA sequences with matching restriction sites with the enzyme DNA ligase.

nonpermuted virus: the order of sequences in a particular virus or vector as found in the original retrovirus in nature, e.g., gag , pol , env without interruption by sequences extraneous to these genes.

oncogenic sequences: genes which have been discovered to encode functions that are capable of transforming cells to an oncogenic state.

phenotype: the gross appearance, behavior or capabilities of an organism that are the observable expression of underlying genetic characteristics, e.g., eye color.

proviral DNA: the deoxyribonucleic acid molecule produced as a result of reverse transcription of the ribonucleic acid genome of a retrovirus; the proviral DNA often undergoes rearrangements and duplication of sequences present in the RNA genome, and is capable of integration into the DNA genome of its host.

reactive expression: the process whereby a given gene is expressed in an cell, organism. or animal, and in which such expression yields a positive influence on the phenotype of said cell, organism, or animal.

receptor: a protein usually localized in the outer membrane of a cell that recognizes a specific ligand with high affinity.

replication: the process whereby a genome is duplicated by DNA polymerase or RNA polymerase

restriction enzyme: an enzyme capable of recognizing a specific local sequence of bases in a DNA duplex and producing a double strand scission at that location; the cut site of the enzyme is convenient for joining pieces of DNA with like cut sites.

retrovirus: an obligate intracellular parasite whose RNA genome is reverse transcribed into a DNA sequence once infection of a host cell has occurred, and may become integrated into the host cell genome as a provirus.

reverse transcriptase: the enzyme encoded by the retroviral pol gene responsible for generation of a proviral DNA copy of the retroviral RNA genome

transfection: the technique for introducing nucleic acid molecules, usually of viral origin, into a cell such that the cell becomes infected subsequently produces virus

transformation: the technique for introducing a plasmid into a bacterial cell; also the description of a cell that has become neoplastic

transgenic animal: an animal carrying in its chromosomal material sequences from a foreign chromosome.

vector: a piece of DNA, often in a circular conformation, capable of replication in an appropriate organism or cell and capable of accepting additional DNA sequences.

## ABBREVIATIONS

ALV: avian leukosis virus
bGH: bovine growth hormone
BGHMT: the bovine growth hormone gene under transcriptional regulation of the mouse metallothionein promoter
BHpol : Bryan High titer polymerase gene
cDNA: complementary DNA copy of a messenger RNA
CEF: chicken embryo fibroblasts
cGH: chicken growth hormone
env : the envelope gene of the retrovirus
gag : the retroviral group-specific antigen
LTR: long terminal repeat
pol : the gene encoding reverse transcriptase
REV: Reticuloendotheliosis virus
RSV: Rous Sarcoma Virus
SR-A: Schmitt Ruppin, A strain of RSV

## DETAILED DESCRIPTION OF THE INVENTION

### A. Vector Construction

This invention relates to retroviral vectors for the production of transgenic fowl. This technology overcomes the inaccessibility of the avian embryo which has to date limited the success of genetic manipulation of the avian genome. In addition, the invention discloses a nonpermuted retroviral vector such that no bacterial or other sequences need to be removed from the construct prior to transfection of a host cell for the production of an infectious virus stock. Therefore less in vitro manipulation results in increaced efficiency as every input molecule of vector results in a transfected cell that produces virus. In addition, inclusion in the vector of a gene encoding a highly efficient polymerase substantially reduces the post-transfection lag time typical in the production of a high titer stock of infectious virus.

As the invention relies on unique features of retroviral physiology, it will be profitable to briefly review the salient features of the retroviral life cycle.

A retrovirus is an obligate intracellular parasite composed of an envelope protein defining its host specificity (env ), a group-specific core antigen (gag ) and a reverse transcriptase enzyme (pol ). The pol gene product is responsible for generating a DNA copy of the RNA genome enclosed within the viral particle composed of the elements described above.

Upon 'docking' of the viral env protein with a receptor present on the surface of a host's cell, the virus penetrates into the cell and the RNA dependent DNA polymerase begins reverse transcription of the viral RNA. The resulting proviral DNA copy inserts itself into the host cell genome and is replicated along with the cell's other genetic material during cell division. The viral proteins are produced as a result of the cellular machinery transcribing integrated viral DNA sequences into messenger RNA which is then

translated by the cell into new copies of gag , env , and pol . Some of the RNA is incorporated into new viral particles which then bud from the cell to begin a new round of infection.

Due to their physiology, retroviruses are in many ways ideally suited to the task of ferrying foreign genes into eukaryotic genomes. They are highly infectious in culture, usually transfecting 100% of a population of cells. They are small and therefore readily manipulated in vitro, and essential functions for the encoded genes have been elucidated.

However, the use of retroviral vectors for introduction of foreign genes has a number of drawbacks. Namely many naturally occurring retroviruses encode oncogenic sequences, for example src in the rous sarcoma virus. It is therefore necessary to eliminate these undesirable sequences. In fact a useful gene might be inserted in place of the removed oncogenic sequences.

Retroviruses are inherently unstable. They have become adept at integrating themselves into the genomes of their hosts, sometimes picking up new sequences, sometimes losing sequences. Many higher eukaryotic genomes are loaded with retroviral sequences integrated in the form of the DNA proviral copy of the viruse's RNA genome. The presence of these genomic proviral copies of the retrovirus in part contributes to the instability of retroviral vectors due to homologous recombination.

The retroviral promoters are encompassed by the long terminal repeats (LTR's), so named because of the identical sequences occurring at either end of the provirus during the DNA phase of its life cycle. The identity of these sequences exacerbates the problems of homologous recombination. Since the LTR's are also such powerful enhancers of gene transcription, they have the added shortcoming of being capable of activating host oncogenic sequences. A solution to these problems may lie in the use of weaker promoters incapable of activating undesirable gene products, and in replacing regions of high homology to edogenous proviruses with sequences of less homology having similar functionality.

An ideal vector would be highly infectious to the target tissue of interest, but should be limited to a single round of infection such that a viremic animal is not produced. Such vectors are deleted for one or more of the genes necessary for multiple rounds of infection. This system however requires the use of a helper virus or cell [Temin et al. US 4,650,764 (1987)] to generate a high titer viral stock for an initial infection. Attendant with this technology is the increased probability of rearrangements including deletion of the desired exogenous gene during passage through the helper cell line. This shortcoming is being adressed, and helper cell lines are emerging in which the incidence of deletions and rearrangements due to homologous recombination is greatly attenuated.

The vector should contain no oncogenic sequences, and should be modified so as to be ineffective in the activation of undesirable genomic sequences. In addition, the vector should insert itself into a known predetermined location in the host chromosome so as to be in a transcriptionally active context, while in no way interrupting host gene functions [Dorin et al., Science 243 : 1357-1360 (1989)].

Finally, the vector should be capable of stably introducing a desired expressible gene into the host chromosome to allow the production of the desired effect in the animal under study.

The state of the art has as yet not met all of these criteria, but rapid advances are being made. The instant invention is a significant step forward in the quest for a retroviral vector delivery system for production of transgenic fowl.

Several different retroviruses have been identified that have adapted themselves to different avian hosts (Hughes et al., Virology 108 : 222-229 (1981); Gudkov et al., J. Gen. Virol. 57 : 85-94 (1981)). In general the genomes of these viruses are constructed along similar functional motifs (see fig. 1). In a linear sequence, one encounters from left to right, a long terminal repeat (LTR) comprising viral replication control elements (promoter, enhancer, integration functions), the structural gene coding region (gag , pol , env ), an oncogenic sequence such as src (not always present), followed by the right hand LTR. In addition, many of the retroviral sequences identified to date have similar restriction enzyme maps.

The procedure for preparing a functional retroviral vector requires the formation of a proviral DNA copy of the RNA, and subsequent modification of the DNA using standard molecular biology techniques (digestion with appropriate restriction enzymes, ligation of sequences of interest, etc. Maniatis et al. "Molecular Cloning: A Laboratory Manual." Cold Spring Harbor Laboratory, Cold Spring Harbor, New York). In general, it is convenient to replace unwanted oncogenic sequences with desirable sequences such as a foreign gene and a transcriptional promoter. It is also suggested that different host specificity may be conferred upon the vector due to the nature of the env gene product. Manipulations have been described in which such modifications have been performed resulting in infection of a foreign cell type by retroviruses derived from another species. In performing these manipulations, it is important not to damage any of the essential viral replication functions unless a replication defective vector is desired (Temin, U.S. 4,650,764)).

The decision as to whether to use a replication competent or a replication defective vector will depend largely on what is planned for the fowl that is ultimately to receive the foreign gene being transferred by the

retroviral vector. Replication competent vectors are simpler to use as they require no additional helper functions, and the ongoing infection they produce obviates the necessity for the presence of a marker gene. On the other hand, transgenio fowl generated by the use of a replication competent vector will be viremic and thus not suitable for commercialization.

Replication defective vectors have been described in the art [Bosselman et al. J. of Virol. 63 : 2680-2689 (1989); Bosselman et al. Science 243 : 533-535 (1989)]. These vectors code for a defective retroviral protein, usually the env gene product, such that the virus requires provision of the defective protein in trans. The production of a high titer stock of such a replication defective recombinant retrovirus thus requires culturing in a cell line that expresses the missing protein. The production of appropriate helper cells is the subject of U.S. patent # 4,650,764 of Temin. The infection of a developing avian embryo with a high titer stock of a replication defective recombinant retrovirus requires infection of as many of the embryonic avian cells as possible upon initial exposure as no additional virus will be generated thereafter. In this case it becomes critical that a high titer stock of recombinant retrovirus carrying the desired foreign gene be used.

As an example the RNA of the Schmidt-Ruppin A strain of the avian Rous Sarcoma retrovirus may be reverse transcribed with reverse transcriptase to form a DNA copy of the viral genome. Upon formation of a second DNA strand, appropriate linkers may be ligated onto the ends of the DNA, and large amounts of the proviral DNA generated by cloning into a vector carrying an E. coli origin of replication (for example pBR322). Such cloned retroviral genomes are known and available in the art (Molecular Biology of Tumor Viruses, 2nd Edition, RNA Tumor Viruses, Cold Spring Harbor Laboratories, 1982). Appropriate deletions, additions and substitutions may then be performed and the resultant proviral DNA used for introduction into tissue culture cells to produce a high titer of infective virus for introduction into the avian germline.

Removal of src may be accomplished by digestion of the proviral DNA, generation of an acceptable cloning site by linker ligation, insertion of a desired sequence with compatible ends, and repair of any deleted essential replication sequences. Desirable sequences, such as a growth hormone gene, might confer enhanced growth characteristics, whereas expression of interferon induced proteins such as the murine MX protein [Horisberger et al. P.N.A.S. U.S.A. 80 : 1910-1914 (1983); Haller et al. WO87/00864: (Feb. 12, 1987)] or of pathogenic viral genes, for example the Newcastle disease virus hemagglutinin-neuraminidase gene, might confer enhanced disease resistance.

It is possible to depend on the viral LTR promoter and enhacer functions to drive expression of the foreign inserted gene sequences. It may however be preferable to insert a foreign promoter adjacent to the desired foreign gene sequence. It would often be most advantageous to utilize a promoter with a known mode of repression or activation. Such promoters of transcription are known in the art, the most commonly utilized of which is the zinc-inducible mouse metallothionein promoter. A number of constitutive promoters whose use has been reported include the SV40 early promoter, the phosphoenolpyruvate carboxykinase promoter, cytomegalovirus immediate early promoter, and the chicken actin gene promoter.

It is desirable to generate a vector that enhances the stability of an inserted foreign gene. Crittendon et al. attempted to accomplish this stabilization by removal of homologous noncoding regions, as in removal of one of the direct repeats, in order to minimize deletion by homologous recombination. In our hands, in vitro experiments, in which viral stocks were produced in chicken embryo fibroblasts, indicated that deletion of desirable sequences continued to occur in vectors modified essentially as described by Crittendon. Our solution to this problem is to construct a vector encoding a more efficient polymerase, in addition to deleting the upstream direct repeat. As shown by Sorge et al., it is necessary to retain the intact downstream direct repeat to allow packaging of retroviral sequences into virions [J. of Virol. 48 : 667-675 (1983)]

By replacing the pol gene of RSV with the gene encoding the Bryan High-titer polymerase (BHpol ), preparation of a high titer viral stock with a minimal amount of in vitro replication and manipulation is facilitated. In addition, a viral stock with the requisite titer may be generated, as a result of this replacement, in roughly half the time required compared with a vector encoding a low titer polymerase. In addition, the construction of nonpermuted vectors means that a minimal amount of in vitro manipulation of the construct is required prior to transfection. It is unnecessary to remove bacterial or other sequences as infectious virus is produced by transfection of the intact construct. These modifications thus minimize recombination and deletion of inserted sequences.

At least five different subclasses of virus have been assigned on the basis of susceptibility of cells from given fowl to infection by the virus: A, B, C, D, E. This specificity occurs as a result of either the presence or absence on the avian cell surface of receptors for the env protein of the infecting virus. The species specificity of the vector may thus be modified by replacement of the env gene with an env gene from a homologous retrovirus that infects the desired species [Dorner et al., J. Virol. 53 : 32-39 (1985); Bova et al., Virology 152 : 343-354 (1986); Kornbluth et al., Mol. Cell Biol. 6 : 1545-1551 (1986); Hughes et al., J. Virol.

61 : 3004-3012 (1987)]. Since many of the homologous retroviruses have emerged from a common ancestor it is often a simple matter to discover convenient restriction sites to facilitate the replacement. For example, the relevant region of env lies within the 1 kilobase Kpn1-Sal1 fragment of pSRpol -beta [Sudol et al. Nuc. Acids Res. 14 : 2391-2405 (1986)]. This segment may conveniently be exchanged with the corresponding region from the retroviral molecular clones of subgroup B and D viruses which have homologous Kpn1 and Sal1 restriction sites (see example 8). Indeed, four out of five of the recognized env subtypes are displayed by isolates of the Schmidt-Ruppin RSV [Molecular Biology of Tumor Viruses 2nd Edition, RNA Tumor Viruses, Cold Spring Harbor Laboratory, (1982)].

## B. Vector Transfection and Generation of a High Titer Viral Stock:

In the generation of a high titer viral stock for infection of the avian egg, it is necessary to transfect cells in vitro with the DNA construct encoding the proviral DNA. There are several commonly used techniques for transfection of eukaryotic cells in vitro. Calcium phosphate precipitation of DNA is most commonly used [Bachetti et al. P.N.A.S 74 : 1590-1594 (1977); Maitland et al. Cell 14 : 133-141 (1977)]. Microinjection of DNA into the pronucleus of the single celled mouse embryo has yielded transgenic mice [Leder et al., U.S., patent 4,736,866; Wagner et al., P.N.A.S. U.S.A. 78 , 5016-5020 (1981)]. Electroporation of DNA into cells is a new technique that is finding increasing popularity [Neumann et al. EMBO L. 1 :841 (1982)]].

The applicants utilized the calcium phosphate method of Cross et al. [Cell 34 :597-607 (1983)] to transfect and culture primary or secondary chicken embryo fibroblasts and found that in one of the preferred embodiments of the retroviral vector (pNPBGH85) that a high titer viral stock could be generated in five days. In addition, quail cells in continuous culture were used to advantage according to Moscovici et al. [Cell 11 : 95-103 (1977)].

Retroviruses will delete foreign sequences during in vitro passage designed to generate the high titer infective stock. The recognition that replacement of the low titer polymerase with a high titer polymerase gene, in addition to deletion of the upstream direct repeat, would contribute to overcoming this shortcoming is not obvious, but we report here that it is one of the possible solutions to this problem. The feasibility of generating the high viral titer of $10^7$ infectious units per milliliter (as compared with about $10^5$ IU/ml for the low titer polymerase vectors) in vitro for infection of the avian egg, within 5 days rather than the usual 2-3 weeks, without deletion or modification of the desired gene of interest, is a significant improvement on existing techniques.

The culture supernatant may be titered for viral stock concentration by a number of methods. We have used either a serial dilution endpoint assay wherein a known volume of 2 fold dilutions of the viral stock being titered is used to infect a culture of chicken embryo fibroblasts. After several days of culture, scoring of culture wells for infected cells either by p27 ELISA or bGH dot blot assay allows computation of the original viral stock concentration. Alternatively, a p27 ELISA may be used to calculate the concentration of viral envelope proteins in culture directly and thus to determine the viral concentration.

A volume of 100 microliters of a stock of $10^7$ infectious units per milliliter represents $10^6$ infectious units. Since the day one avian embryo consists of between

$$10^4 \text{ and } 5\text{x}10^5$$

cells, introduction of $10^6$ infectious viral particles means that whether or not a replication competent vector is used, every cell of the developing blastoderm, including the germ-line cells, will stand an excellent chance of undergoing infection by this initial viral assault.

## C. Infection of the Day One Avian Egg:

As the avian embryo is only to be found in the single celled stage prior to ovaposition one method for introducing a desired foreign gene into the developing fowl is to laboriously remove the avian ovuum, use one of the above described techniques for transfection, and reimplant the transfected ovuum [Shunman et al. Poult. Sci. 65 : 1436-1444 (1986); Kopchick et al., "Methods for the Introduction of Recombinant DNA into Chicken Embryos," to be published in Transgenic Technology in Medicine and Agriculture, F.H. Haseltine and N. First (Eds.), National Institute of Health, Bethesda, MD]. However, recent efforts using retroviral vectors have demonstrated the feasibility of infecting the day one avian egg by introduction of retroviruses directly under the developing blastoderm.

The day one avian egg is wiped with an ethanol swab at the large air sack end of the egg and a small hole is drilled into the shell with a sterile bit. A volume of virus between 10 and 300 microliters, depending on the viral stock concentration is injected into the egg just below the blastoderm. The hole is wiped with an ethanol swab and resealed with wax. The egg is then incubated under conditions kown in the art to promote development of the avian egg to term.

Use of a sufficiently high titer viral stock for introduction into the avian blastoderm makes infection of germline as well as somatic cells highly likely, in spite of the large number of cells in the day one egg.

The vector, pNPBGH85, was used to transform E. coli DH5 and the culture given the name MB5436. This culture was deposited under the Budapest Treaty with the ATCC on October 4, 1989 and given ATCC accession number 68106. Another vector, pNPRAV, was used to transform E. coli DH5 and the culture given the name MB5437. This culture was deposited under the Budapest Treaty with the ATCC on October 4, 1989, and given ATCC accession number 68107. The ATCC adress is: 12301 Parklawn Dr., Rockville, MD 20852.

The following examples are provided to delineate specific embodiments of the invention but are not intended to limit the scope of the claims that follow:

## EXAMPLE I

Construction of pNPBGH85 (figure 2), carrying the bovine growth hormone gene, and generation of a high titer viral stock:

Step I: Vector Preparation

Step A. Preparation of pSRBGH85:

1) A 2.9 kilobase Kpn I fragment was isolated from the bovine growth hormone expression vector pBGH-10, of Kelder, et al. Gene 76 , 75-80, (1989), incorporated here by reference for these purposes. The fragment was blunt ended with the klenow enzyme using standard molecular biology techniques

2) The 12 kilobase Cla I fragment from Schmidt-Ruppin RSV subgroup A was isolated and blunt ended, according to Hughes et al. Virology 136 , 89-99, (1984).

3) The fragments from 1) and 2) were ligated and the vector pSRBGH85 was recovered from ampicillin resistant E . coli DH5, and shown to have the indicated structure by restriction enzyme mapping (see fig. 1). Note that the metallothionein promoter-bovine growth hormone insert is in an antisense orientation relative to the viral structural genes. As described in example 2, when this vector was digested with SalI and religated to remove pBR sequences and transfected into chicken embryo fibroblasts it was shown to be biologically active: virus was recoverable in culture supernatant.

Step B. Preparation of pSRBGH85XD:

1) pSRBGH85 was digested to completion with Xho I. The reaction was heat-inactivated and diluted to a DNA concentration of 100 ng/ml. Ligated DNA was used to transform E . coli DH5 to ampicillin resistance. One of the resultant clones, pSRBGH85XD, was deleted for the 4.5 kilobase Xho I fragment.

Step C. Preparation of pNPBGH85:

1) pSRBGH85XD was digested to completion with Sal I and EcoR V, and the 4.7 kilobase Sal I fragment containing the retroviral sequences was purified.

2) pSR-pol-beta, of Sudol et al. Nucleic Acids Res. 14 , 2391-2405, (1986) herein incorporated by reference for these purposes, was digested with Sal 1 and and the 10 kilobase fragment was isolated and treated with calf intestinal phosphatase to prevent self-ligation.

3) Fragments from 1) and 2) were ligated and E. coli DH5 was transformed to ampicillin resistance. Restriction mapping of several isolated clones yielded one clone, pNPBGH85, that had the desired nonpermuted proviral DNA structure (i.e. contiguous LTR-gag-BH-pol-env-BGHMt- LTR).

Step II: Transfection of Chicken Embryo Fibroblasts and Virus Preparation:

To test the biological activity of DNA constructs, an in vitro transfection assay of chicken embryo fibroblasts (CEF's) was established as follows:

1) Cells were cultured and transfected according to Cross et al. Cell 34 , 597-607, (1983), herein incorporated by reference for these purposes. One microgram of test DNA was used per 60mm culture dish.

2) Transfected cells were passaged every three to five days.

3) Virus (cell-free supernatant) was harvested from fully infected cultures and used to infect fresh cultures of secondary chicken embryo fibroblasts.

4) Extent of viral infection was monitored by p27gag ELISA (using a Spafas p27 ELISA for avian lymphoid leukosis virus), and was found to be maximal by 5-7 days, although virus could be detected as early as one day post transfection.

5) Culture supernatants were assayed for secretion of bovine growth hormone by a double antibody radioimmunoassay according to Kopchick et al. DNA 4 , 23-31, (1985), hereby incorporated by reference for these purposes. Maximal bovine growth hormone levels occurred at approximately the same time as peak virus production.

6) CEF's transfected with pNPBGH85 and pNPBGH85 itself were each deposited at the American Type Culture Collection, [12301 Parklawn Drive, Rockville, Maryland 20852] on or before the date of filing of the present application.

## EXAMPLE 2

Construction of a Replication Defective bGH Vector, pNPBGHdef (figure 3):

A replication defective vector for producing nonviremic chickens expressing the bovine growth hormone gene is produced by construction of a vector that is deleted for the env gene such that no viral particles can be made inside a cell unless the cell provides the missing env gene product. The construction of such a replication defective vector may be performed in several steps as follows:

Step A : The 9 kilobasepair Sal1-Kpn1 fragment from pSR-pol-beta [Sudol et al. Nuc. Acids Res. 14 : 2391-2405 (1986)] is isolated.

Step B : The 220 basepair Kpn1-Pst1 fragment from pBH-beta [Lerner and Hanafusa, J. Virol. 49 : 549-556 (1984)] is isolated.

Step C: The 3.9 kilobasepair partial Pst1-Sal1 fragment from pSRBGH85XD (see example 1) is isolated.

Step D: Preparation of pNPBGHdef:

In a three-part ligation mix, the elements from A, B, and C are joined. E. coli is transformed with ligated DNA and a clone carrying a plamid with the desired structure, pNPBGHdef,obtained. The vector is comprised of LTR-gag -BHpol -BGHMT-LTR sequences, similar to the genome of defective Bryan RSV, with the bGH gene replacing the src gene. This vector is expected to generate transformed chicken embryo fibroblasts, and whole fowl, expressing bGH at the same levels obtained for pNPBGH85 (examples 5 and 6).

## EXAMPLE 3

Construction of pNPRAV, a Replication Competent Helper Virus Vector:

In order to utilize the replication defective vector, pNPBGHdef of example 2, it is necessary to transfect cells in culture, and produce, thereby, a viral stock. As pNPBGHdef is missing a functional env gene, the envelope protein must be provided in trans. This may be accomplished by transfecting a cell previously engineered to produce envelope glycoprotein [Temin et al. U.S. 4,650,764 (1987)]. Alternatively, a cell may be co-transfected with a vector that carries a functional env gene, such that the pNPBGH85 RNA is packaged into the pNPRAV env gene product. Construction of a helper virus vector, pNPRAV, was

performed as follows:

Step A: Preparation of pSRXDCla:

The subgroupA Schmidt-Ruppin RSV derived vector p779/989-1089/2795 [Hughes and Kosik, Virology 136 : 89-99 (9184)] was digested to completion with Xho1. The reaction mixture was heat-inactivated and diluted to a DNA concentration of 100 ng/ml. DNA was ligated and used to transform E. coli DH5 to ampicillin resistance. One resultant clone , pSRXDCla, was found to have deleted the 4.5 kilobasepair Xho1 fragment. Thus, pSRXDCla contains a unique Cla1 cloning site into which foreign genes can be inserted. Such inserts reside in the position that the removed src gene usually occupies in the RSV genome.

Step B. Preparation of pNPRAV:

pSRXDCla was digested to completion with Sal1 and EcoRV, and the 2.9 kilobasepair Sal1 fragment containing the retroviral sequences was isolated. This fragment was ligated to Sal1 linearized and phosphatase treated pSR-pol-beta [Sudol et al. Nuc. Acids Res. 14 : 2391-2405, (1986)]. Ligated DNA was used to transform DH5 E. coli to ampicillin resistance. One resultant clone, pNPRAV, was found to have the desired structure, comprising the nonpermuted proviral DNA of an avian leukosis virus LTR-gag-pol-env-LTR. Upon transfection into chicken embryo fibroblasts, pNPRAV produces infectious replication-competent virus, and can be used as a helper to complement a replication-defective virus.

## EXAMPLE 4:

Stable expression of bovine growth hormone in pNPBGH85-Transfected CEF's:

The vector pNPBGH85 was prepared as described supra. (example 1) pNPRAV, a plasmid which produces a replication competent helper virus was prepared in a multistep procedure analogous to that used for the production of pNPBGH85 but with no inserted foreign gene (example 2). Secondary chicken embryo fibroblasts were transfected by calcium phosphate precipitation as described (example 1), using either no DNA, pNPRAV, or pNPBGH85. Transfected cultures were passaged every three to five days for a total of 25 days. Every five days, cell-free supernatant was harvested from confluent cultures and assayed for bovine growth hormone by radioimmunoassay. Genomic DNA was prepared from parallel transfected cultures.

Expression of bovine growth hormone was specific to pNPBGH85-transfected cells and was stable during long-term culture of fully infected cells. DNA dot blot analysis of genomic DNA verified the presence of bovine growth hormone sequences (using a bovine growth hormone-specific probe derived from pBGH-10) and integrated provirus (using an exogenous LTR specific probe).

Presence of virus in culture fluid was verified by p27gag ELISA analysis of cell lysates of fresh chicken embryo fibroblasts infected with virus harvested from transfected cells. Virus titer was determined by endpoint dilution titration and p27gag ELISA.

TABLE I

| Average Bovine Growth Hormone Concentration (Duplicate Samples) | | | | | |
|---|---|---|---|---|---|
| | bGH Concentration, ng/ml | | | | |
| Days post Transfection: | 5 | 10 | 15 | 20 | 25 |
| DNA Transfected: | | | | | |
| None | 3.8 | 4.9 | 5.7 | 2.1 | 4.5 |
| pNPRAV | 3.6 | 5.7 | 4.0 | 2.2 | 4.4 |
| pNPBGH85 | 149.6 | 227.0 | 179.0 | 182.8 | 183.5 |

## EXAMPLE 5

Construction of a Vector Carrying a Foreign Gene Other than the Bovine Growth Hormone Gene. PNPluc:

The methodology utilized for the construotion of pNPBGH85 and pNPBGHdef may be generalized for the construction of avian retroviral vectors carrying other foreign genes. This example describes the construction of pNPluc and pNPlucdef, replication competent and replication defective vectors respectively, carrying the firefly luciferase gene.

Step I Preparation of pNPluc:

Step A. Preparation of pSRlucd:

pSRXDCla (see example 4 supra) was digested to completion with the Cla1 restriction enzyme, blunt ended, and treated with phosphatase. The 1.8 kilobase HindIII-partial Xba1 fragment from the luciferase expression vector pSV232AL-Ad5′ [DeWet et al. Mol. Cell. Biol. 7 : 725-737 (1987)] containing the complete cDNA for firefly luciferase was blunt ended, and ligated to the 7.3 kilobasepair blunt ended Cla1 pSRXDCla vector. Ligated DNA was used to transform E. coli DH5 and a clone with the desired structure, pSRlucd, was isolated. The luciferase cDNA is in the same transciptional orientation as the retroviral structural genes in this construct.

Step B. Preparation of pNPluc:

pSRlucd was digested to completion with SalI and BglI and the 4.7 kilobasepair SalI fragment containing the retroviral sequences was purified. This fragment was ligated to the phosphatase treated 10 kilobasepair pSR-pol-beta SalI fragement. Ligated DNA was used to transform E. coli DH5, and a clone isolated, pNPluc, having the desired structure: a nonpermuted proviral sequence with the luciferase gene replacing src . Expression of the luciferase gene in this vector is mediated by the viral transcriptional control signals, ie. the LTR promoter, the gag splice donor site, src acceptor site, and 3′ polyadenylation signal.

Step II Construction of a Replication Defective Luciferase Vector. pNPlucdef:

A replication defective luciferase vector lacking envelope coding sequences was constructed by a three

part ligation of the following DNA fragments:

A : 3.9 kilobasepair PstI-SalI pSRlucd (see above, example 5) fragment.

B : 220 basepair Kpn1-Pst1 pBH-beta [Lerner et al. J. Virol. 49 : 549-556 (1984)] fragment.

C : 9 kilobasepair SalI-KpnI fragment from pSR-pol-beta.

Ligated DNA was used to transform E. coli DH5 and a clone with the desired structure, pNPlucdef, was isolated.

## EXAMPLE 6

Biological Activity of Luciferase Vectors:

To assay the biological activity of the luciferase vector constructs, secondary chicken embryo fibroblasts were transfected with plasmid DNA as previously described (see example 1 supra). Transfected cells produced virus with the predicted proviral structures, as analyzed by Southern blot hybridization using either luciferase or LTR-specific probes. Cell lysates were prepared and analyzed for luciferase activity as described [DeWet et al. Mol. Cell. Biol. **7**: 725-737 (1987)]. Both pNPluc and pNPlucdef (when cotransfected with pNPRAV) produce virus carrying the luciferase gene, and both produce equivalent amounts of luciferase activity.

TABLE II

| Long Term Expression of Luciferase Activity | | | | | |
|---|---|---|---|---|---|
| | Day Post-Transfection | | | | |
| DNA Transfected: | 5 | 10 | 15 | 20 | 25 |
| none | 24 | 34 | ND | ND | ND |
| pNPRAV | 37 | 39 | 51 | 48 | 38 |
| pNPluc | 149,714 | 152,997 | 149,031 | 153,601 | 125,748 |
| pNPBGH85 | 12 | 156 | 41 | 52 | 34 |

TABLE III

| Biological Activity of Luciferase Vectors | |
|---|---|
| DNA Transfected | Luciferase Activity |
| None | 63 |
| pNPluc | 155,243 |
| pNPRAV | 49 |
| pNPlucdef | 270 |
| pNPlucdef + pNPRAV 1:1 | 41,846 |
| pNPlucdef + pNPRAV 2:1 | 110,531 |
| pNPlucdef + pNPRAV 5:1 | 164,774 |

## EXAMPLE 7

Introduction of the Non-Permuted, High Titer Vector pNPBGH85 into Chicken Eggs:

Fertilized eggs from Hubbard Leghorn line 139 chickens were collected immediately after laying and stored with the large-end up at 15° C until use. A small portion of the egg shell (5 mm by 5 mm) was removed from the top without disturbing the inner membrane. After localizing the blastoderm, 50 ul volumes of virus stock, titered at $10^7$ infectious units per milliliter, were injected in the immediate area of the blastoderm using a 27 gauge needle connected to a micropipet. Following this manipulation, the opening in the shell was covered with cellophane tape and sealed with paraffin. Eggs were incubated at 38° C for further development. After hatching the chickens, meconium samples were taken for detecting virus by p27 ELISA. Blood samples were also obtained for DNA dot blot of high molecular weight DNA and serum bGH radioimmunoassay. Results from one experiment are provided in table IV. The two males, 9667 and 9818 are closely observed and the following physiological effects were noted: 1) Both males began semen production at about 13 weeks instead of at the normal age of 16 to 18 weeks. 2) Both males had longer legs 20.2 cm and 20.1 cm at 22 weeks as compared with the average of 17.5 cm for control birds and many of the other transgenics of the same strain at 39 weeks. 3) At 26 weeks, these fowl weighed 6.1 lb, and 5.5 lb, as compared with the average weight of 4.4 lb for control birds. Fifteen other birds expressed bGH at various levels, however, physiological changes were not apparent in these fowl. No progeny were produced from these birds, however it is clear from the work of Bosselman et al. (Science 243 : 533-535 (1989)) that once foreign sequences have become integrated into the avian genome, these sequences will be transmitted to progeny in a mendelian fashion.

TABLE IV

| Bird # | Sex | Virus Assay | DNA (copies per cell) Viral | bGH | Serum bGH (ng/ml) | Age (Wks) |
|---|---|---|---|---|---|---|
| 9667 | M | + | 2 | 1 | 97 | 4 |
| | | | | | 76 | 18 |
| 9818 | M | + | 2 | 1 | 123 | 4 |
| | | | | | 86 | 35 |
| 146 | F | + | 2 | 0.2 | 23 | 4 |
| | | | | | 38 | 32 |
| 195 | M | + | 1 | 1 | 20 | 4 |
| | | | | | 8 | 31 |
| 309 | M | + | 2 | 1 | 61 | 4 |
| | | | | | 25 | 28 |
| 332 | F | + | 0.5 | 0.5 | 55 | 4 |
| | | | | | 55 | 27 |
| 450 | M | + | 1 | 1 | 46 | 4 |
| | | | | | 38 | 26 |
| 612 | M | + | 1 | 0.5 | 49 | 4 |
| | | | | | 80 | 21 |
| 646 | M | + | 2 | 0.1 | 48 | 4 |
| | | | | | 40 | 21 |
| 710 | M | + | 1 | 0.1 | 36 | 4 |
| | | | | | 43 | 21 |
| 878 | F | + | 1 | 0.1 | 16 | 4 |
| | | | | | 11 | 19 |
| 911 | M | + | 0.5 | 0.5 | 24 | 4 |
| | | | | | 19 | 19 |

TABLE IV (continued)

| Bird # | Sex | Virus Assay | DNA (copies per cell) Viral | bGH | Serum bGH (ng/ml) | Age (Wks) |
|---|---|---|---|---|---|---|
| 987 | M | + | 1 | 0.5 | 18 | 4 |
| | | | | | 10 | 18 |
| 12-514 | ND | + | 1 | 0.1 | 60 | 4 |
| 12-561 | ND | + | 1 | 0.2 | 283 | 3 |
| 12-568 | ND | + | 1 | 0.2 | 49 | 3 |
| 12-582 | ND | + | 1 | 0.1 | 20 | 3 |

## Example 8

Vector Constructs Carrying an env Gene Other than Subgroup-A:

Vectors carrying the subgroup-A env gene may be restricted in to infection of hosts expressing the subtype-A receptor on their cellular surface. As mentioned above (vector construction), due to the homology of avian retroviral genomes, it is possible to remove a restriction fragment from a given env gene subtype and replace it with a restriction fragment from another viral subtype env gene and thereby confer a new host specificity on the vector.

Step 1 : pSRpol -beta is digested to completion with Kpn1 and Sal1, and the 9 kilobase fragment is isolated.

Step 2 : a) Isolate the 1 kilobase Kpn1-Sal1 restriction fragment from pRAV2 [Ju et al., J. Virol. 33 : 1026-1033 (1980)], which is a molecular clone specifying the subtype B env .

b) Isolate the Kpn1-Sal1 restriction fragment from λSRD-RSV-D-4 [Shalloway et al., Cell 24 : 531-541 (1981)] a molecular clone of a virus carrying the subgroup D env gene.

Step 3 : Ligate the fragment from step 1 with either the fragment from step 2a or step 2b to obtain a vector with a subgroup B or D env gene respectively. The ligation mixture is used to transform E. coli and a clone with the desired substitution in env is selected.

In the case of replication defective vectors, a helper with no foreign gene but encoding a functional env gene product with the desired subgroup would provide the same specificity as the vectors described in this example.

## Claims

1. A nonpermuted retroviral vector encoding the Bryan High Titer Polymerase and the bovine growth hormone gene as a foreign expressible gene.

2. The retroviral vector of Claim 1 comprising the genes gag , pol , env , the LTR, at least one direct repeat sequence, integration, replication, and control elements necessary for infection of avian cells.

3. The retroviral vector of Claim 2 in which the source of viral sequences is the Schmidt Ruppin A strain of Rous Sarcoma virus having the src oncogene, in which the direct repeat upstream from the src oncogene has been removed to prevent deletion of the bovine growth hormone gene, where said bovine growth

hormone gene replaces the src oncogene, and where the env gene is either the Schmidt Ruppin A strain gene or has been deleted or replaced by an env conferring infectivity on a different avian species.

4. The vector of Claim 3 in which the bovine growth hormone gene is preceeded by a transcriptional promoter.

5. A nonpermuted avian retroviral vector comprising the gene encoding the bovine growth hormone under transcriptional control of the mouse metallothionein promoter, the Rous Sarcoma Virus structural genes env , gag , and pol , the pol gene being of the Bryan High Titer variety, the long terminal repeats, at least one direct repeat, and additional sequences as necessary to provide replication competence to said vector.

6. The retroviral vector of Claim 5 which is pNPBGH85.

7. The vector of Claim 6 in which the Rous Sarcoma virus structural gene env is either deleted or replaced by an env gene conferring infectivity on a different avian species.

8. The retroviral vector of Claim 7 which is pNPBGHdef.

9. The retroviral vector of Claim 8 from which the bovine growth hormone gene has been deleted such that no expressible foreign gene is present in the vector, allowing for the insertion of a foreign expressible gene other than the bovine growth hormone gene.

10. The retroviral vector of Claim 9 which is pNPRAV.

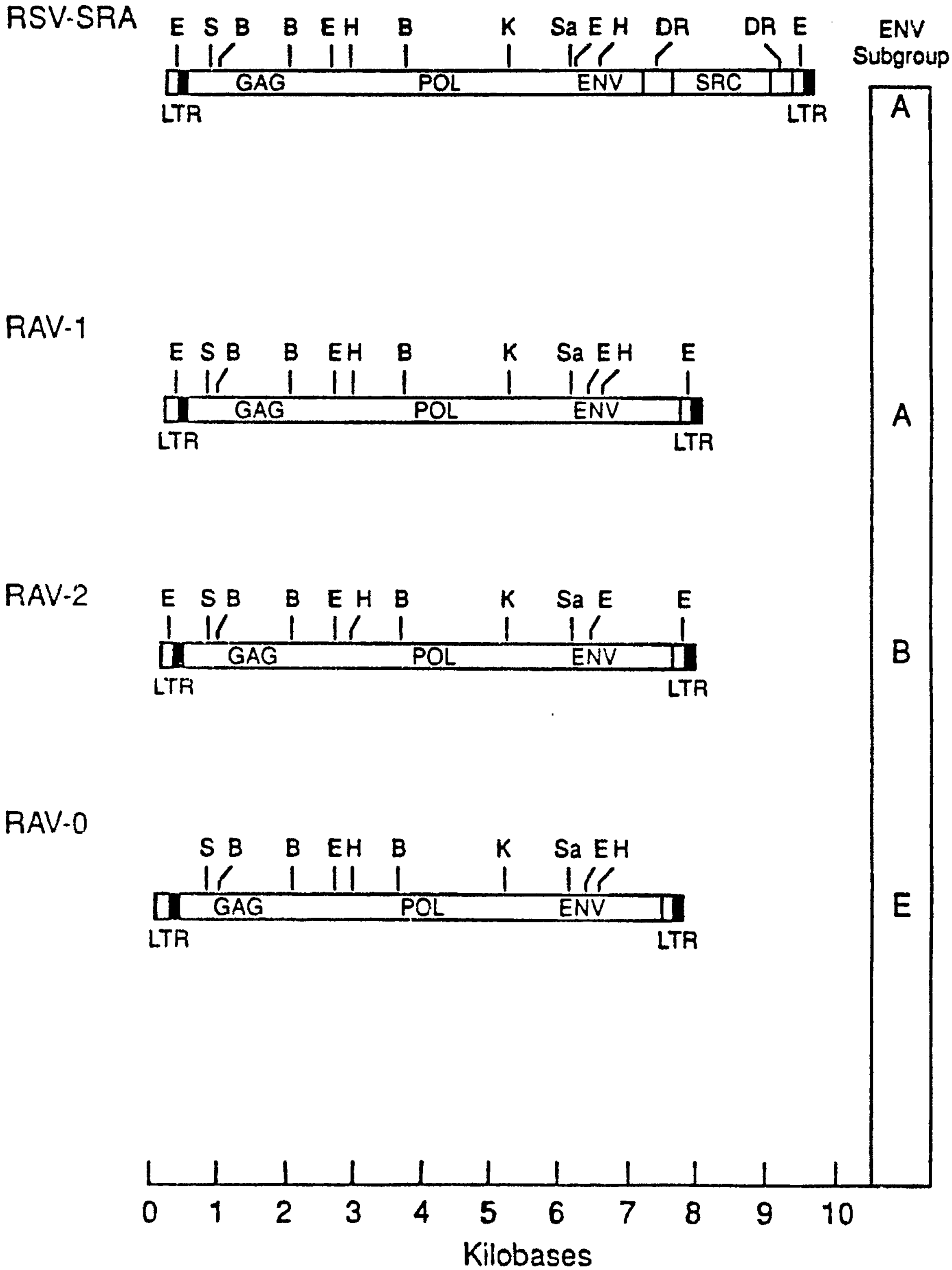


Abbreviations: S=Sst, Sa=Sal I, H=Hind III, K=Kpn I, B=BamHI, E=EcoRI

*FIG. 1*

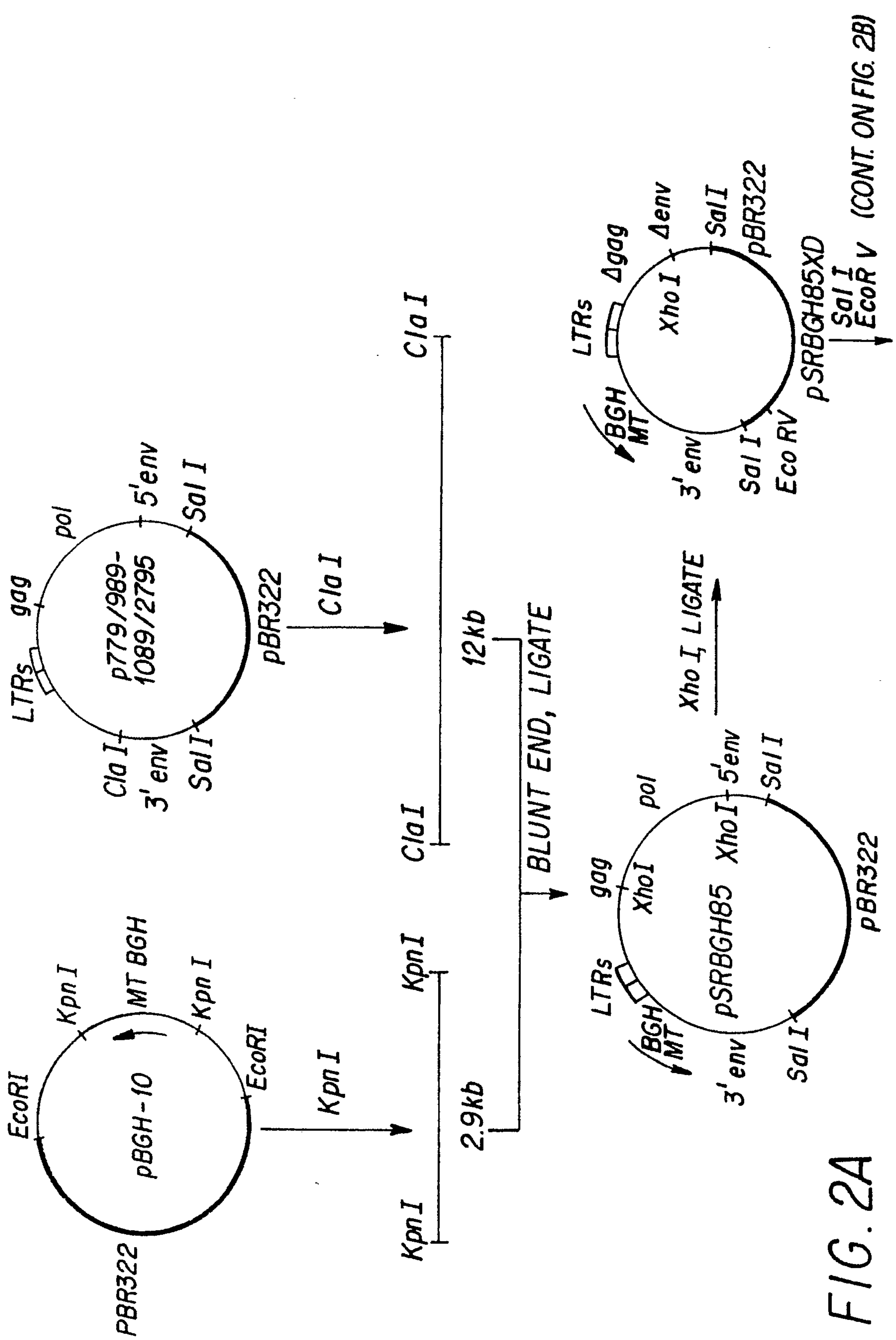
EcoRI
KpnI
MT BGH
KpnI
EcoRI
pBGH-10
PBR322
KpnI
KpnI
2.9kb
KpnI
LTRs
gag
pol
5'env
SalI
p779/989-
1089/2795
pBR322
ClaI
3'env
SalI
ClaI
ClaI
12kb
ClaI
BLUNT END, LIGATE
LTRs
gag
XhoI
pol
XhoI
5'env
SalI
BGH
MT
pSRBGH5
3'env
SalI
pBR322
XhoI, LIGATE
LTRs
Δgag
Δenv
XhoI
SalI
pBR322
BGH
MT
3'env
SalI
EcoRV
pSRBGH5XD
SalI
EcoR V
(CONT. ON FIG. 2B)

FIG. 2A

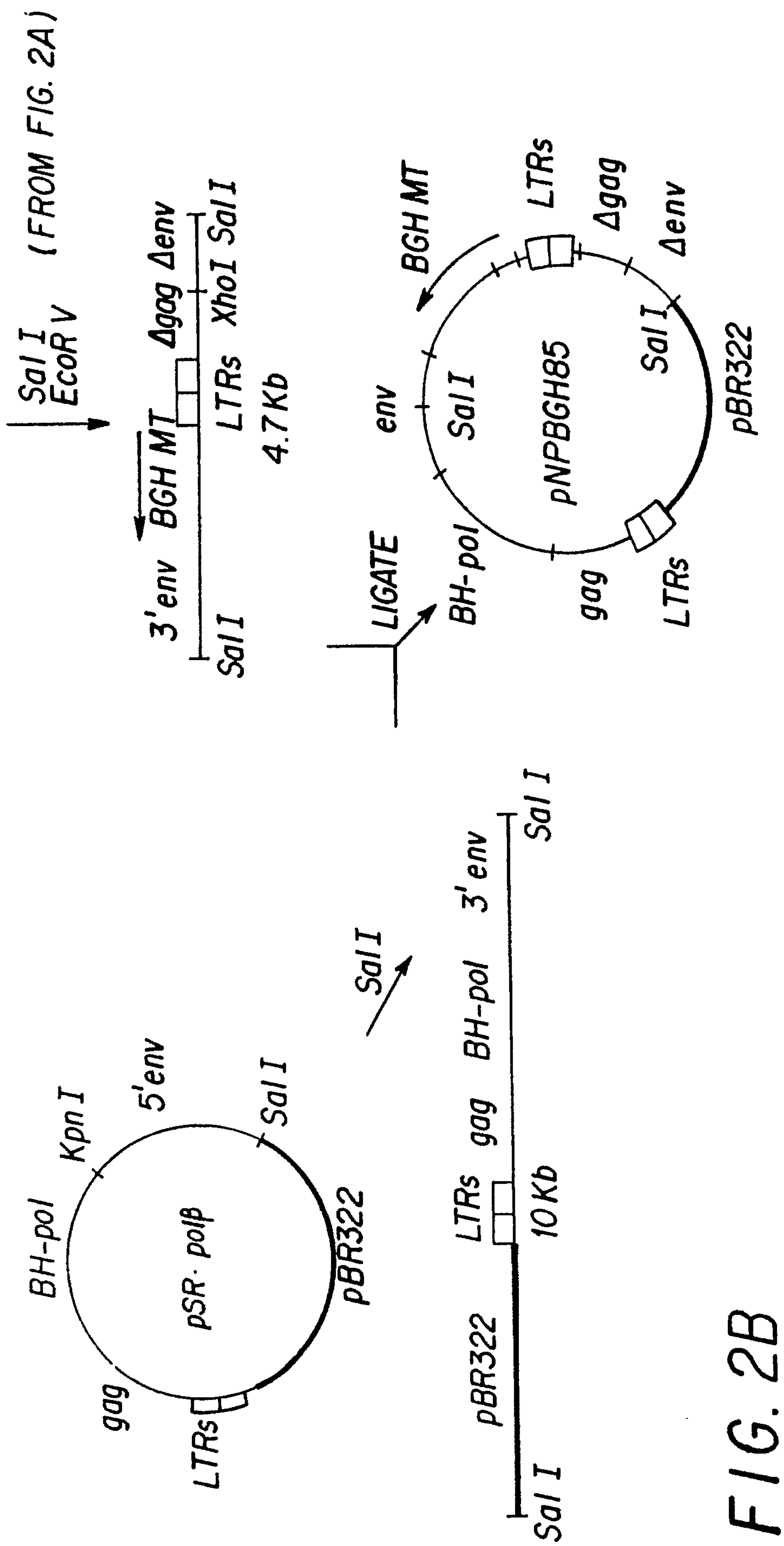
(FROM FIG. 2A)
Sal I
EcoR V
BGH MT
3'env
Sal I
LTRs
4.7Kb
Δgag
Δenv
XhoI
Sal I
LIGATE
BH-pol
gag
LTRs
env
Sal I
pNPBGH85
Sal I
pBR322
BGH MT
LTRs
Δgag
Δenv
BH-pol
Kpn I
5'env
Sal I
pSR · polβ
pBR322
gag
LTRs
Sal I
pBR322
LTRs
gag
10 Kb
BH-pol
3'env
Sal I
Sal I

FIG. 2B

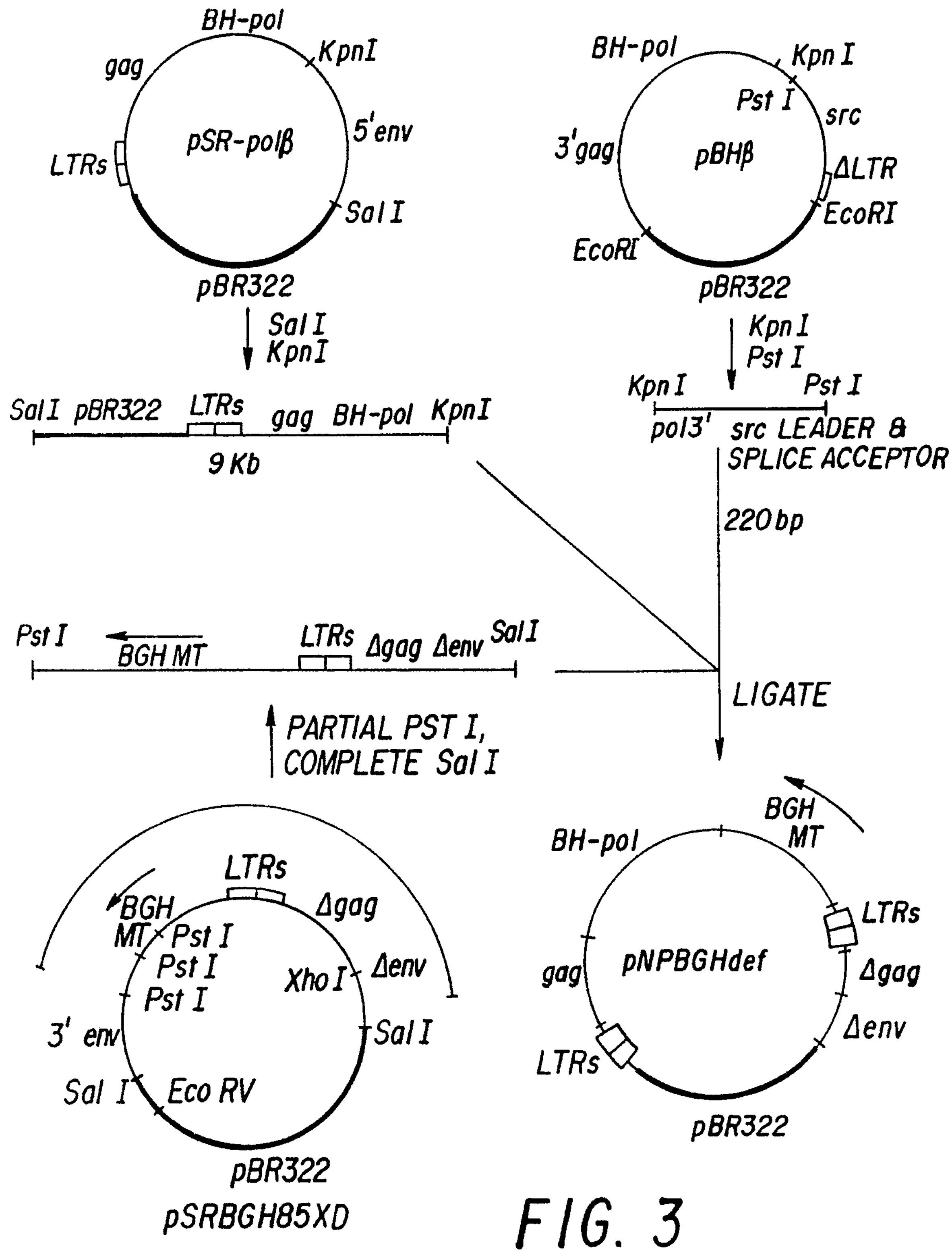
BH-pol
KpnI
gag
pSR-polβ
5'env
LTRs
SalI
pBR322
BH-pol
Kpn I
Pst I
src
3'gag
pBHβ
ΔLTR
EcoRI
EcoRI
pBR322
SalI
KpnI
Kpn I
Pst I
Kpn I
Pst I
SalI pBR322 LTRs gag BH-pol KpnI
9 Kb
pol3' src LEADER &
SPLICE ACCEPTOR
220 bp
Pst I
BGH MT
LTRs Δgag Δenv
SalI
LIGATE
PARTIAL PST I,
COMPLETE SalI
LTRs
BGH
MT
Pst I
Δgag
Pst I
XhoI
Δenv
Pst I
3' env
SalI
SalI
Eco RV
pBR322
pSRBGH85XD
BGH
MT
BH-pol
LTRs
gag
pNPBGHdef
Δgag
Δenv
LTRs
pBR322

FIG. 3

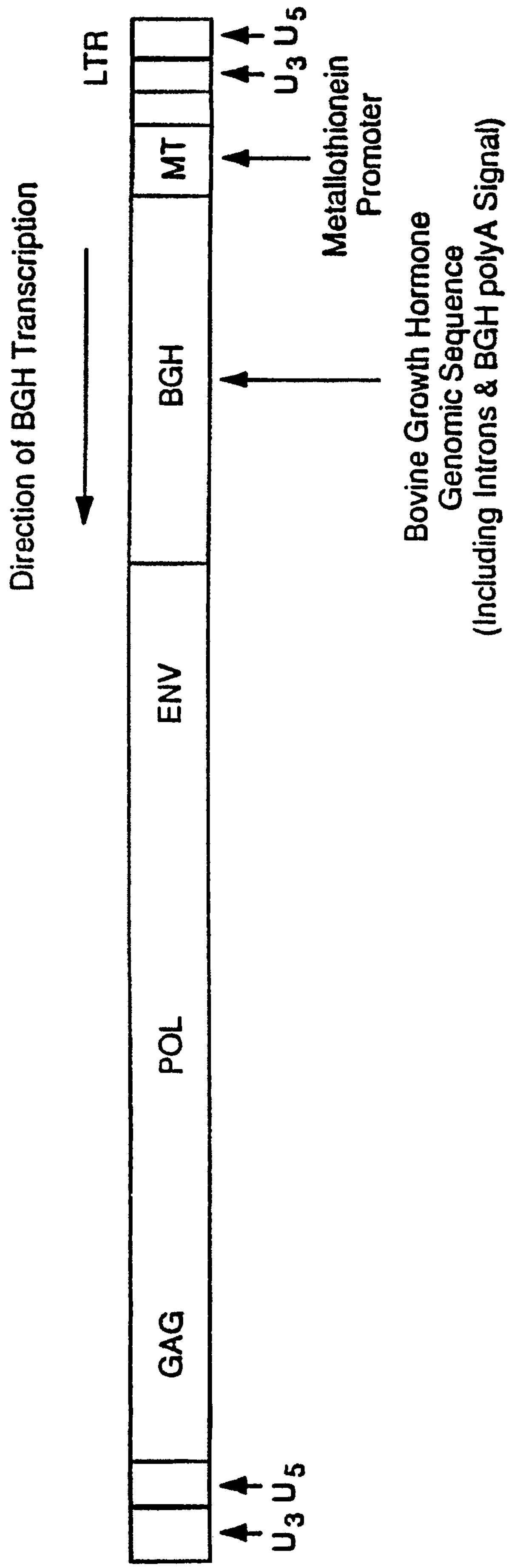
Direction of BGH Transcription
LTR
MT
BGH
ENV
POL
GAG
$U_3$ $U_5$
$U_3$ $U_5$
Metallothionein Promoter
Bovine Growth Hormone Genomic Sequence (Including Introns & BGH polyA Signal)

FIG. 4

European Patent Office

# EUROPEAN SEARCH REPORT

Application Number

EP 90 31 1149

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | 18th Annual Meeting of UCLA: Symp. Mol. Cell. Biol., J. Cell. Biochem. vol. suppl, no. B, 03 February 1989, page 178 CHEN, H.Y. et al.: "Expression of the bovine Growth Hormone gene in transgenic chickens" * page 178, paragraph 206 * --- | 1-10 | C12N15/00<br>C12N15/86 |
| Y | GENE vol. 76, no. 1, 1989, pages 75 - 80; KELDER, B. et al.: "Aactivation of the mouse methallothionein-I promoter in transiently transfected avian cells" * the whole document * --- | 1-10 | |
| Y | GENE vol. 38, no. 1-3, 1985, AMSTERDAM NL pages 227 - 232; PASLEAU, F. et al.: "Growth Hormone gene expression in eukaryotic cells directed by the Rous sarcoma virus long terminal repeat or cytomegalovirus immediate-early promoter" * the whole document * ----- | 1-10 | TECHNICAL FIELDS SEARCHED (Int. Cl.5 )<br>C12N |

The present search report has been drawn up for all claims

| Place of search | Date of completion of the search | Examiner |
|---|---|---|
| THE HAGUE | 18 JANUARY 1991 | CHAMBONNET F.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)